# EUROPEAN PATENT SPECIFICATION

(11) **EP 2 863 923 B1**
(45) Date of publication and mention of the grant of the patent: **08.05.2019**
(21) Application number: 13730912.6
(22) Date of filing: 25.06.2013
(51) Int. Cl.: A61K 31/5517, A61K 35/00

(54) **THIENOTRIAZOLODIAZEPINE COMPOUNDS FOR THE TREATMENT OF LYMPHOMAS**
THIENOTRIAZOLODIAZEPIN-VERBINDUNGEN ZUR BEHANDLUNG VON LYMPHOMEN
COMPOSES DE THIÉNOTRIAZOLODIAZÉPINE POUR LE TRAITEMENT DE LYMPHOMES

(30) Priority: 25.06.2012 US 201261663885 P; 12.07.2012 US 201261670918 P
(43) Date of publication of application: 29.04.2015
(73) Proprietor: Oncoethix GmbH, 6006 Luzern (CH)
(72) Inventor: BERTONI, Francesco, CH-6500 Bellinzona (CH); BONETTI, Paola, CH-6500 Bellinzona (CH)
(74) Representative: Jaap, David Robert
(86) International application number: PCT/EP2013/063313
(87) International publication number: WO 2014/001356

(56) References cited:
- EP-A1- 2 239 264
- WO-A1-2013/033420
- WO-A2-2011/143669

## Description

### FIELD OF THE INVENTION

The present invention relates to a composition for use in treating B-cell malignant cancers and T-cell malignant cancers as defined in the appended claims.

### BACKGROUND OF THE INVENTION

Bromodomain-containing proteins play an important role in gene expression regulation, via chromatin structure remodelling. Antitumor activity has been reported in acute and chronic hematological malignancies, including B-cell and T-cell malignancies, using inhibitors of BRD2/3/4, members of the Bromodomain and Extraterminal (BET) family. B-cell malignancies, which are also known as B-cell neoplasms or B-cell lymphomas, are cancers that occur when B-cells are overproduced or are malignant. B-cell malignancies include for example diffuse large B-cell lymphoma (DLBCL), mantel cell lymphoma (MCL), splenic marginal zone lymphoma (SMZL), and multiple myeloma (MM). T-cell malignancies, such as anaplastic large T-cell lymphoma, are a heterogeneous group of lymphoid neoplasms representing malignant transformation of the T lymphocytes. The use of thienotriazolodiazepines in the treatment of cancer, e.g., Burkitt's lymphoma is disclosed in EP 2239264. Furthermore, WO2011/143669 discloses thienotriazolodiazepine compound JQ-1 for the treatment of neoplasms, such as cutaneous T-cell lymphoma (CTCL), and lymphoma (Hodgkin's disease, non-Hodgkin's disease).

The present disclosure presents compositions for use in treating certain B-cell malignant cancers and T-cell malignant cancers.

### SUMMARY OF THE INVENTION

The present application is defined in the appended claims. Subject matters which are not encompassed by the scope of the claims do not form part of the present invention.

In one embodiment, the invention provides for a composition for use in treating B-cell malignant cancers or T-cell malignant cancers, said composition comprising a thienotriazolodiazepine compound represented by Formula 1: 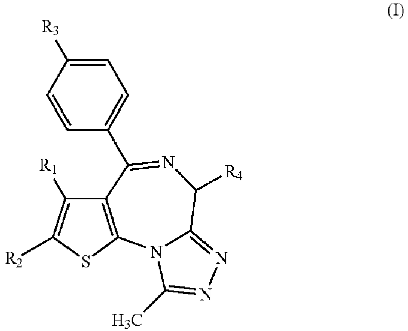 wherein R₁ is alkyl having a carbon number of 1-4, R₂ is a hydrogen atom; a halogen atom; or alkyl having a carbon number of 1-4 optionally substituted by a halogen atom or a hydroxyl group, R₃ is a halogen atom; phenyl optionally substituted by a halogen atom, alkyl having a carbon number of 1-4, alkoxy having a carbon number of 1-4 or cyano; --NR₅--(CH₂)ₘ--R₆ wherein R₅ is a hydrogen atom or alkyl having a carbon number of 1-4, m is an integer of 0-4, and R₆ is phenyl or pyridyl optionally substituted by a halogen atom; or --NR₇--CO--(CH₂)ₙ--R₈ wherein R₇ is a hydrogen atom or alkyl having a carbon number of 1-4, n is an integer of 0-2, and R₈ is phenyl or pyridyl optionally substituted by a halogen atom, and R₄ is --(CH₂)ₐ--CO--NH--R₉ wherein a is an integer of 1-4, and R₉ is alkyl having a carbon number of 1-4; hydroxyalkyl having a carbon number of 1-4; alkoxy having a carbon number of 1-4; or phenyl or pyridyl optionally substituted by alkyl having a carbon number of 1-4, alkoxy having a carbon number of 1-4, amino or a hydroxyl group or --(CH₂)_{b}-COOR₁₀ wherein b is an integer of 1-4, and R₁₀ is alkyl having a carbon number of 1-4, or a pharmaceutically acceptable salt thereof or a hydrate or solvate thereof

In one embodiment, the present invention provides for a composition for use in treating B-cell malignant cancers or T-cell malignant cancers, said composition comprising a thienotriazolodiazepine compound independently selected from the group of (i) (S)-2-[4-(4-chlorophenyl)-2,3,9-trimethyl-6H-thieno[3,2-f][1,2,4]triazolo- [4,3-a][1,4]diazepin-6-yl]-N-(4-hydroxyphenyl)acetamide or a dihydrate thereof, (ii) methyl (S)-{4-(3'-cyanobiphenyl-4-yl)-2,3,9-trimethyl-6H-thieno[3,2-f][1,2,4]tri- azolo[4,3-a][1,4]diazepin-6-yl} acetate, (iii) methyl (S)-{2,3,9-trimethyl-4-(4-phenylaminophenyl)-6H-thieno[3,2-f][1,2,4]triaz- olo[4,3-a][1,4]diazepin-6-yl}acetate; and (iv) methyl (S)-{2,3,9-trimethyl-4-[4-(3-phenylpropionylamino)phenyl]-6H-thieno[3,2-f- ][1,2,4]triazolo[4,3-a][1,4]diazepin-6-yl} acetate.

In one embodiment, the present invention provides for a composition for use in treating B-cell malignant cancers or T-cell malignant cancers, said composition comprising (S)-2-[4-(4-chlorophenyl)-2,3,9-trimethyl-6H-thieno[3,2-f][1,2,4]triazolo-[4,3-a][1,4]diazepin-6-yl]-N-(4-hydroxyphenyl)acetamide having the structure of Formula 2:

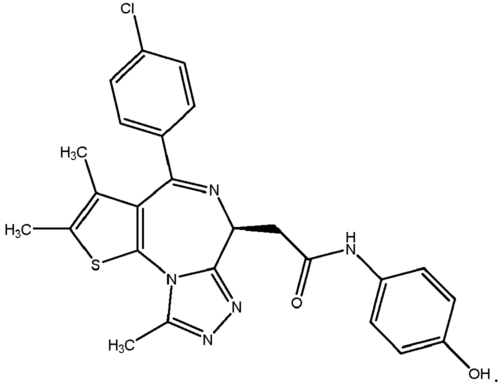

In one embodiment, the present invention provides for a composition for use in treating B-cell malignant cancers or T-cell malignant cancers in a patient, wherein said composition comprises a thienotriazolodiazepine compound represented by Formula 1 and wherein the patient is a human.

In one embodiment for treating B-cell malignant cancers using a pharmaceutically acceptable amount of Formula (1), the B-cell malignant cancers include diffuse large B-cell lymphoma and splenic marginal zone lymphoma. In another embodiment treating T-cell malignant cancers using a pharmaceutically acceptable amount of Formula (1), the T-cell malignant cancers include anaplastic large T-cell lymphoma.

In one embodiment, the present invention provides for a composition for use in treating B-cell malignant cancers or T-cell malignant cancers in a patient, said composition comprising (S)-2-[4-(4-chlorophenyl)-2,3,9-trimethyl-6H-thieno[3,2-f][1,2,4]triazolo-[4,3-a][1,4]diazepin-6-yl]-N-(4-hydroxyphenyl)acetamide having the structure of Formula 2:

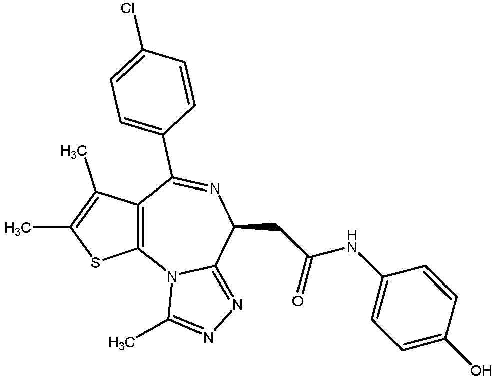

In one embodiment, the present invention provides for a composition for use in treating B-cell malignant cancers or T-cell malignant cancers in a patient by administering to a patient, said composition comprising a thienotriazolodiazepine compound represented by Formula 2 wherein the patient is a human.

In one embodiment for treating B-cell malignant cancers using a pharmaceutically acceptable amount of Formula (2), the B-cell malignant cancers include diffuse large B-cell lymphoma and splenic marginal zone lymphoma. In another embodiment for treating T-cell malignant cancers using a pharmaceutically acceptable amount of Formula (2), the T-cell malignant cancers include anaplastic large T-cell lymphoma.

### BRIEF DESCRIPTION OF THE DRAWINGS

The foregoing summary, as well as the following detailed description of the invention, will be better understood when read in conjunction with the appended drawings.
Figure 1 illustrates cell cycle alterations induced by various concentrations of Formula 2 in DLBLC cell lines, DoHH₂, U-2932, Karpas 422, SU-DHL-6 and Val. X-axis, cell lines. Y-axis, percentage of cells in each cell cycle phase.
Figures 2A-2C illustrate the induction of cellular senescense in DoHH₂ DLBLC cell line and L-82 ALCL cell line after 48 hours exposure to Formula 2. Y-axis is percentage of cells positive to β-galactosidase.
Figures 3A nd 3B illustrate the expression levels of BRD2, BRD3 and BRD4 in DLBCL cell lines, SU-DHL-2, SU-DHL-4, SU-DHL-5, SU-DHL-6, SU-DHL-7, Val, OCI-Ly7, U-2932, DoHH₂ and Karpas 422. X-axis, cell lines. Y-axis, mRNA quantities, relative to GAPDH.
Figures 4A and 4B illustrate the expression levels of BRD2, BRD3 and BRD4 in ALCL cell lines, MAC1, FE-PD, Karpas 299, SU-DHL-1, SUPM-2, L82, JB6 and TS. X-axis, cell lines. Y-axis, mRNA quantities, relative to GAPDH.
Figure 5A-5F illustrate MYC mRNA levels after increasing doses of Formula 2 in DLBCL cell lines, SU-DHL-2, OCI-Ly3, U-2932, DoHH₂, Karpas 422 and SU-DHL-6. X-axis, cell lines. Y-axis, mRNA quantities, relative to un-treated sample.
Figures 6A-6D illustrate MYC mRNA levels after increasing doses of Formula 2 in ALCL cell lines, L82, Karpas 299, FE-PD and SU-DHL-1. X-axis, cell lines. Y-axis, mRNA quantities, relative to un-treated sample.
Figures 7A-7C illustrate MYC mRNA levels for DLBCL cell lines, DoHH₂, Karpas 422 and SU-DHL-2, after 2 hour exposure of 1 µM Formula 2 followed by wash-out.
Figures 8A-8C illustrate the effect of Formula 2 on the proliferation of DLBCL cell lines, DoHH₂, U-2932 and SU-DHL-6, with time after 24 hour treatment with IC50 dose of Formula 2 followed by wash-out.
Figures 9A-9B illustrate NFκB targets mRNA levels (IRF4, A20, BIRC3) in ABC-DLBCL cell lines, SU-DHL-2 and U-2932, after increasing doses of Formula 2. X-axis, cell lines. Y-axis, fold change, relative to un-treated sample.

### DETAILED DESCRIPTION OF THE INVENTION

In one embodiment, the invention provides for a composition for use in treating B-cell malignant cancers or T-cell malignant cancers, said composition comprising a thienotriazolodiazepine compound. In one such embodiment, the thienotriazolodiazepine compound is represented by the following Formula (1): 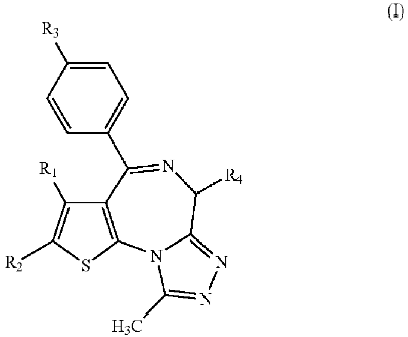 wherein R₁ is alkyl having a carbon number of 1-4, R₂ is a hydrogen atom; a halogen atom; or alkyl having a carbon number of 1-4 optionally substituted by a halogen atom or a hydroxyl group, R₃ is a halogen atom; phenyl optionally substituted by a halogen atom, alkyl having a carbon number of 1-4, alkoxy having a carbon number of 1-4 or cyano; --NR₅--(CH₂)ₘ--R₆ wherein R₅ is a hydrogen atom or alkyl having a carbon number of 1-4, m is an integer of 0-4, and R₆ is phenyl or pyridyl optionally substituted by a halogen atom; or --NR₇CO--(CH₂)ₙ--R₈ wherein R₇ is a hydrogen atom or alkyl having a carbon number of 1-4, n is an integer of 0-2, and R₈ is phenyl or pyridyl optionally substituted by a halogen atom, and R₄ is --(CH₂)ₐ--CO--NH--R₉ wherein a is an integer of 1-4, and R₉ is alkyl having a carbon number of 1-4; hydroxyalkyl having a carbon number of 1-4; alkoxy having a carbon number of 1-4; or phenyl or pyridyl optionally substituted by alkyl having a carbon number of 1-4, alkoxy having a carbon number of 1-4, amino or a hydroxyl group or --(CH₂)_{b}-COOR₁₀ wherein b is an integer of 1-4, and R₁₀ is alkyl having a carbon number of 1-4, or a pharmaceutically acceptable salt thereof or a hydrate or solvate thereof. In one such embodiment, the patient is a human.

Also disclosed is a composition for use in treating B-cell malignant cancers or T-cell malignant cancers wherein said composition comprises a thienotriazolodiazepine compound represented by Formula (1) and wherein the B-cell malignant cancers or T-cell malignant cancers is independently selected from Hodgkin's lymphoma or non-Hodgkin's lymphoma. In one such embodiment, the patient is a human.

In one such disclosure, the Hodgkin's lymphoma is independently selected from nodular sclerosis classical Hodgkin's lymphoma (CHL), mixed cellularity CHL, lymphocyte-depletion CHL, lymphocyte-rich CHL and nodular lymphocyte predominant Hodgkin's lymphoma.

In another such disclosure, the non-Hodgkin's lymphoma is independently selected from AIDS-related lymphomas, anaplastic large-cell lymphoma, angioimmunoblastic lymphoma, blastic NK-cell lymphoma, chronic lymphocytic leukemia/small lymphocyti lymphoma, cutaneous T-cell lymphoma, diffuse large B-cell lymphoma, eteropathy-type T-cell lymphoma, follicular lymphoma, hepatosplenic gamma-delta T-cell lymphoma, lymphoblastic lymphoma, marginal zone lymphoma, nasal T-cell lymphoma, pediatric lymphoma, peripheral T-cell lymphomas, primary central nervous system lymphoma, T-cell leukemia, transformed lymphomas, treatment-related T-cell lymphoma and Waldenstrom's macroglobulinemia.

In one embodiment, the present invention provides for a composition for use in treating diffuse large B-cell lymphoma in a patient, said composition comprising a thienotriazolodiazepine compound represented by Formula 1. In one such embodiment, the patient is a human.

In one embodiment, the present invention provides for a composition for use in treating splenic marginal zone lymphoma in a patient by administering to a patient, said composition comprising a thienotriazolodiazepine compound represented by Formula 1. In one such embodiment, the patient is a human.

In one embodiment, the present invention provides for a composition, for use in treating anaplastic large T-cell lymphoma in a patient, said composition comprising a thienotriazolodiazepine compound represented by Formula 1. In one such embodiment, the patient is a human.

In another embodiment the invention comprises a composition for use in treating B-cell malignant cancers or T-cell malignant cancers in a patient, said composition comprising a thienotriazolodiazepine compound represented by Formula 1 that is selected from the group consisting of: (i) (S)-2-[4-(4-chlorophenyl)-2,3,9-trimethyl-6H-thieno[3,2-f][1,2,4]triazolo- [4,3-a][1,4]diazepin-6-yl]-N-(4-hydroxyphenyl)acetamide or a dihydrate thereof, (ii) methyl (S)-{4-(3'-cyanobiphenyl-4-yl)-2,3,9-trimethyl-6H-thieno[3,2-f][1,2,4]tri-azolo[4,3-a][1,4]diazepin-6-yl}acetate, (iii) methyl (S)-{2,3,9-trimethyl-4-(4-phenylaminophenyl)-6H-thieno[3,2-f][1,2,4]triazolo[4,3-a][1,4]diazepin-6-yl} acetate; and (iv) methyl (S)-{2,3,9-trimethyl-4-[4-(3-phenylpropionylamino)phenyl]-6H-thieno[3,2-f-][1,2,4]triazolo[4,3-a][1,4]diazepin-6-yl} acetate. In one such embodiment, the patient is a human.

In another embodiment the invention comprises a composition for use in treating B-cell malignant cancers or T-cell malignant cancers in a patient, said composition comprising (S)-2-[4-(4-chlorophenyl)-2,3,9-trimethyl-6H-thieno[3,2-f][1,2,4]triazolo-[4,3-a][1,4]diazepin-6-yl]-N-(4-hydroxyphenyl)acetamide (Formula 2), also known as Y-803 and OTX-015: 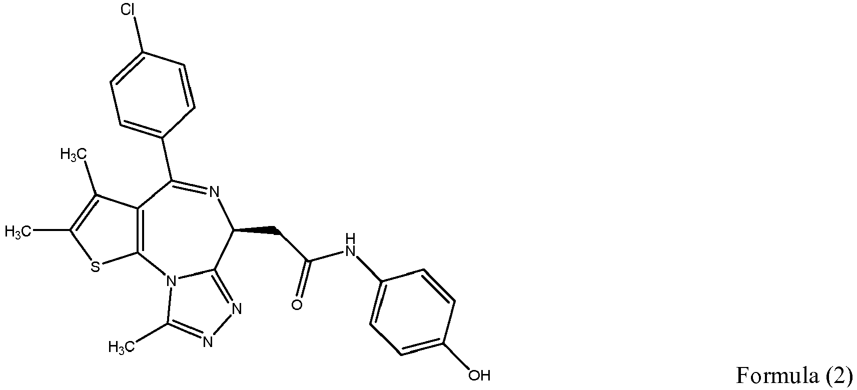 In one such embodiment, the patient is a human.

Also disclosed is a composition for use in treating B-cell malignant cancers or T-cell malignant cancers, said composition comprising a thienotriazolodiazepine compound represented by Formula (2) wherein the B-cell malignant cancers or T-cell malignant cancers is independently selected from Hodgkin's lymphoma or non-Hodgkin's lymphoma. In one such embodiment, the patient is a human.

In one such disclosure, the Hodgkin's lymphoma is independently selected from nodular sclerosis classical Hodgkin's lymphoma (CHL), mixed cellularity CHL, lymphocyte-depletion CHL, lymphocyte-rich CHL and nodular lymphocyte predominant Hodgkin's lymphoma.

In another such disclosure, the non-Hodgkin's lymphoma is independently selected from AIDS-related lymphomas, anaplastic large-cell lymphoma, angioimmunoblastic lymphoma, blastic NK-cell lymphoma, chronic lymphocytic leukemia/small lymphocyti lymphoma, cutaneous T-cell lymphoma, diffuse large B-cell lymphoma, eteropathy-type T-cell lymphoma, follicular lymphoma, hepatosplenic gamma-delta T-cell lymphoma, lymphoblastic lymphoma, marginal zone lymphoma, nasal T-cell lymphoma, pediatric lymphoma, peripheral T-cell lymphomas, primary central nervous system lymphoma, T-cell leukemia, transformed lymphomas, treatment-related T-cell lymphoma and Waldenstrom's macroglobulinemia.

In another embodiment, the invention comprises a composition for use in treating diffuse large B-cell lymphoma in a patient, said composition comprising a thienotriazolodiazepine compound represented by Formula 2. In one such embodiment, the patient is a human.

In one embodiment, the present invention provides for a composition for use in treating splenic marginal zone lymphoma in a patient, said composition comprising a thienotriazolodiazepine compound represented by Formula 2. In one such embodiment, the patient is a human.

In one embodiment, the present invention provides for a composition for use in treating anaplastic large T-cell lymphoma in a patient, said composition comprising a thienotriazolodiazepine compound represented by Formula 2. In one such embodiment, the patient is a human.

The preparation of the compounds represented by Formula 1 and Formula 2 can be accomplished by chemical synthesis by those of ordinary skill in the art according to the methods previously described in the art, including those described in U.S. Patent No. 5,712,274.

The compounds represented by Formula 1 or Formula 2 can be mixed with pharmaceutically-acceptable carriers for oral delivery. The carriers can include binders, lubricants, disintegrants, and other functional and non-functional excipients.

The dose of the compounds represented by Formula 1 or Formula 2 can be determined by one of ordinary skill in the art by taking into consideration the body mass, age, health condition, diet, and other relevant factors presented by a patient as well as the bioavailability of Formula 1 or Formula 2 and the Formula 1 or Formula 2 product formulation. In one embodiment, the oral dose of compound of Formula 1 or Formula 2 may range from 40 to 100 mg.

### EXAMPLES

### EXAMPLE 1: EFFECTS OF FORMULA 2 ON LYMPHOMA CELL PROLIFERATION

The antiproliferative activity of Formula 2 was evaluated in twenty-two (22) diffuse large B-cell lymphoma (DLBCL), eight (8) anaplastic large T-cell lymphoma (ALCL), four (4) mantle cell lymphoma (MCL), and three (3) splenic marginal zone lymphoma (SMZL) established human cell lines. Cell lines were cultured in RPMI-1640 (GIBCO Invitrogen, Basel, Switzerland) or DMEM (GIBCO Invitrogen, Basel, Switzerland) medium supplemented with 10% fetal calf serum, 1% L-glutamine, and penicillin-streptomycin-neomycin (∼5,000 units penicillin, 5 mg/mL streptomycin, 10 mg/mL neomycin).

For proliferation assays, cells were seeded into 96-well plates at a density of 10⁴ cells per well. Formula 2 (OncoEthix SA, Lausanne, Switzerland) was dissolved in DMSO as a stock solution of 10 mM, then divided into aliquots and stored at -80°C. For each experiment, an aliquot of the stock solution was thawed, diluted serially into culture medium, and used within 2 to 3 days. Cells were treated with DMSO (control) or increasing doses of Formula 2 (in five replicates) for 72 hours at 37°C. To detect cell proliferation, 3-(4,5-dimethylthiazol-2-yl)-2,5-diphenyltetrazolium bromide (MTT) (Sigma, Buchs, Switzerland) was prepared as a stock solution of 5 mg/mL in phosphate-buffered saline (PBS) and filter-sterilized. An amount of MTT solution equal to 0.5 mg/mL was then added to each well and incubated in the dark at 37°C for 4 hours. Cells were then lysed with 25% sodium dodecylsulfate (SDS) lysis buffer and absorbance was read at 570 nm on a Beckman-Coulter AD340 instrument. The doses corresponding to the concentration that produced 50% growth inhibition (GI₅₀) were estimated by fitting a sigmoidal model through the dose response curve using the R statistical package (R: A Language and Environment for Statistical Computing, R Foundation for Statistical Computing, Vienna, Austria).

Results, summarized in Table 1, showed that 68% (15/22) of DLBCL lines, 100% (3/3) of SMZL lines, 62% (5/8) of ALCL lines, but no (0/3) MCL lines were sensitive to growth inhibition by Formula 2, where sensitivity was defined by GI₅₀ < 500 nM. Of interest, there was no apparent difference in sensitivity between cell lines derived from DLBCL of the germinal center type (GBC-DLBCL) and those derived from DLBCL of the activated B-cell like type (ABC-DLBCL).

**Table 1: Effects of Compound 2 on Proliferation of Human Lymphoma Cell Lines**

| Lymphoma Subtype | Cell Line | GI₅₀ (nM) | Cell Death, basal/treated (%) | Lymphoma Subtype | Cell Line | GI₅₀ (nM) |
|---|---|---|---|---|---|---|
| Diffuse large B-cell lymphoma, activated B-cell like subtype (ABC-DLBCL) | SU-DHL-2 | 69 | 23 / 87 | Anaplastic large T-cell lymphoma (ALCL) | L82 | 36 |
| | TMD8 | 131 | 51 / 75 | | FE-PD | 158 |
| | OCI-Ly3 | 179 | 7 / 49 | | MAC1 | 311 |
| | U2932 | 202 | 7 / 5 | | Karpas 299 | 411 |
| | OCI-Ly10 | 380 | 7 / 4 | | SUPM2 | 546 |
| | HBL1 | 704 | 2 / 2 | | T5 | 1173 |
| | RIVA | 2280 | 6 / 11 | | JB6 | 1944 |
| | RCK8 | >10,000 | 12 / 10 | | SU-DHL-1 | 9109 |
| Diffuse large B-cell lymphoma, germinal center subtype (GBC-DLBCL) | SU-DHL-10 | 77 | 26 / 32 | | | |
| | DoHH2 | 90 | 16 / 11 | Mantle cell lymphoma (MCL) | Rec-1 | 1224 |
| | OCI-Ly2 | 92 | 6 / 6 | | MAVER-1 | 1224 |
| | SU-DHL-6 | 110 | 9 / 10 | | Jeko-1 | 2787 |
| | SU-DHL-7 | 132 | 5 / 9 | | Granta 519 | >10,000 |
| | OCI-Ly19 | 170 | 14 / 14 | | | |
| | SU-DHL-5 | 189 | 1 / 6 | Splenic marginal zone lymphoma (SMZL) | V151 | 105 |
| | Karpas 422 | 277 | 13 / 5 | | K1718 | 165 |
| | OCI-Ly8 | 527 | 15 / 13 | | SSK41 | 240 |
| | WSU-DLCL2 | 552 | 2 / 1 | | | |
| | SU-DHL-4 | 607 | 5 / 9 | | | |
| | OCI-Ly7 | 1387 | 14 / 27 | | | |
| | OCI-Ly1 | 1550 | 1 / 1 | | | |
| | VAL | >10,000 | 7 / 17 | | | |

| | | | | | | |
|---|---|---|---|---|---|---|
| GI₅₀: Concentration which inhibited proliferation of 50% of cells | | | | | | |

To examine the possible cytotoxic effect of Formula 2 on DLBCL cell lines, the degree of cell death was evaluated after exposure to the compound for 72 hours at doses ranging from 100 to 1500 nM (covering the range of GI₅₀ values). To assess for cell death, cells were treated with DMSO or different doses of Formula 2 for 72 hours, harvested, washed once with PBS, and then stained with propidium iodide (1 µg/mL, Sigma) in PBS. Absorbance was read at 535 nm on a Beckman-Coulter AD340 instrument. The analysis of percentage of cell death was performed using CellQuest Pro software (Becton Dickinson). Results (Table 1) showed that Formula 2 induced cell death in only a small percentage of DLBCL lines with low GI₅₀ values (SU-DHL-2, TMD8, OCI-Ly3).

Additionally, the effect of Formula 2 on induction of apoptosis was evaluated in four DLBCL cell lines (Karpas 422, SU-DHL-2, SU-DHL-6, U2932) and four ALCL cell lines (FE-PD, K299, L82, SU-DHL-1). Cells were treated with DMSO (control) or different doses of Formula 2 for 72 hours, then stained with Click-iT EdU Flow Cytometry Assay Kits (Invitrogen) and 7-ADD (BD Pharmingen) and analyzed for DNA content using a FACScan Flow Cytometer. The analysis of percentage apoptosis was performed using FlowJo 7.6.3 software (Cytek Development, Fremont, California, USA). Results showed no induction of apoptosis in either DLBCL or ALCL cell lines.

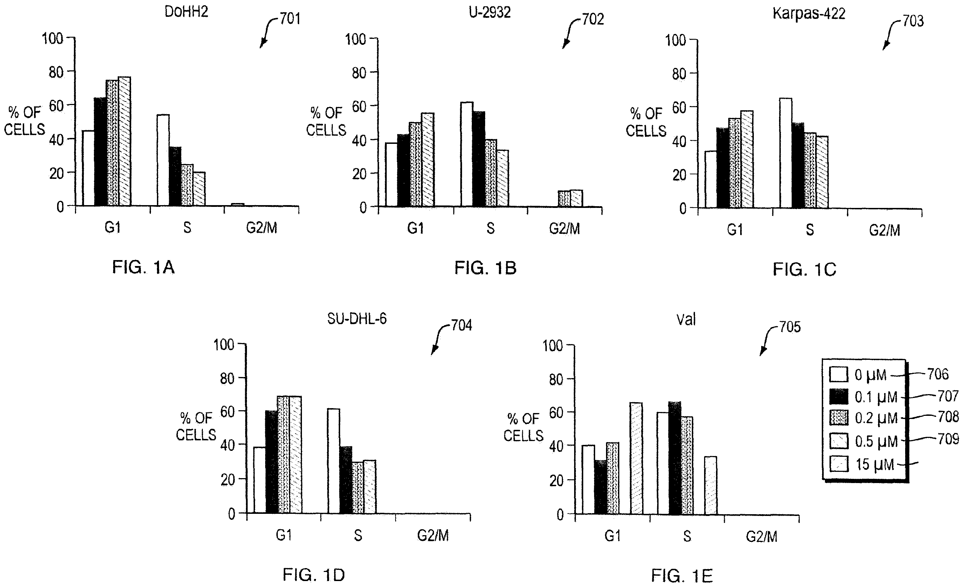

Since Formula 2 did not induce massive cell death or apoptosis despite markedly reducing cell viability, the effect of Formula 2 on cell cycle was evaluated in 5 DLBCL cell lines (DohH2, Karpas 422, SU-DHL-6, VAL, U-2932). Cells were treated with DMSO (control) or different doses of Formula 2 for 24 hours, then harvested, washed once in PBS and fixed in 80% ethanol at 4°C for at least one hour. Fixed cells were stained with propidium iodide (50 µg/mL, Sigma) in PBS containing RNase-A (75 kU/mL, Sigma) and analyzed for DNA content using a FACScan flow cytometer (Becton Dickinson). Cell cycle analysis was performed using the ModFit LT software package (Verity Software House, Inc., Topsham, Maine, USA). As illustrated in Figure 1, the results showed that Formula 2 induced G1-arrest in a dose-dependent manner.

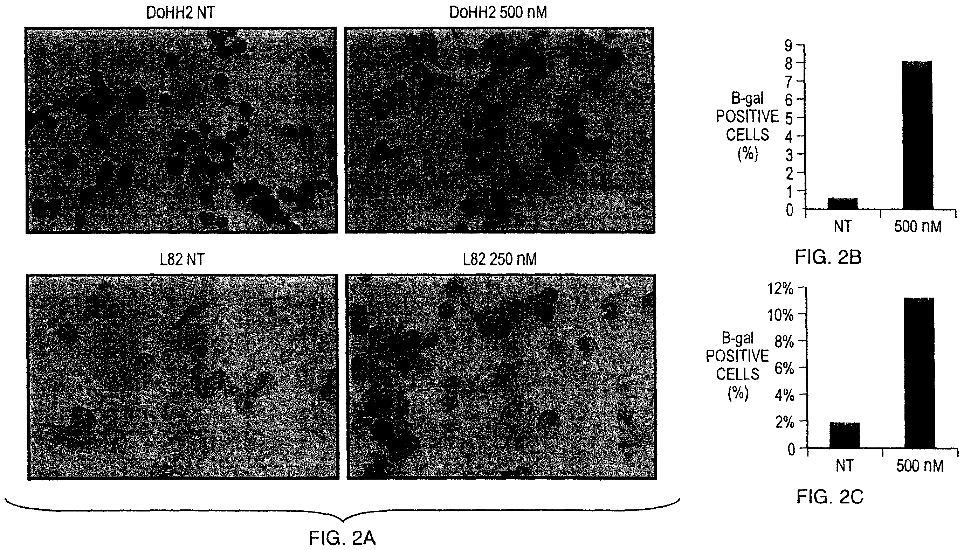

Because Formula 2 induced a marked decrease in cell viability and G1-arrest without induction of apoptosis, induction of cell senescence was evaluated in a representative DLBCL and ALCL cell line. Cells were treated with Formula 2 for 48 hours and then stained for galactosidase. As illustrated in Figure 2, the results showed a marked increase in senescent cells in both the DLBLC and ALCL cell lines suggesting that Formula 2 has mainly a cytostatic effect on lymphoma cells.

### EXAMPLE 2: EFFECTS OF FORMULA 2 ON DOWN-REGULATION OF c-MYC ONCOGENE

Formula 2 previously has been shown to be a competitive inhibitor of the BET bromodomain proteins 2, 3, and 4 (BRD2, BRD3, BRD4) as disclosed in U.S. Patent Appl. Publ. No. 20100286127. Inhibition of BRD4 has been shown to result in down-regulation of the c-MYC oncogene *[*Delmore JE, Issa GC, LemieuxME, et al: BET bromodomain inhibition as a therapeutic strategy to target c-Myc. Cell 2011; 146:1-14*].* Accordingly, basal levels of BRD2, BRD3, and BRD4 mRNA and protein and effects of Formula 2 on c-MYC mRNA and protein levels were evaluated in selected DLBCL and ALCL cell lines.

To assess protein levels, Western blotting analysis was performed as follows. Cells were solubilized in hot SDS lysis buffer (2.5% SDS in pH 7.4 Tris-HCl) and sonicated for 15 seconds. The protein content in the different samples was determined using a bicinchonininic acid (BCA) protein assay (Pierce Chemical Co., Rockford, Illinois, USA). Lysates (40 µg) were fractionated by SDS-PAGE using 8% polyacrylamide gels, based upon the expected molecular weight. The resolved proteins were blotted onto a nitrocellulose membrane by electric transfer. The membranes were blocked in TBS-T buffer (20 mM Tris-HCl, pH 7.6 containing 137 mM NaCl, 0.1% Tween 20, and 5% bovine serum albumin) for one hour. Membranes were then incubated overnight with primary antibodies diluted in TBS-T. The following antibodies were used: anti-BRD2 (ab37633, AbCam, Cambridge, UK), anti-BRD3 (ab56342, AbCam), anti-BRD4 (ab75898, AbCam), and anti-GAPDH (MAB374, Millipore, Billerica, Massachusetts, USA). Membranes were washed in TBS-T three times for ten minutes each and then incubated for one hour in TBS-T containing the appropriate horseradish peroxidase-conjugated anti-mouse or anti-rabbit secondary antibodies (Amersham Life Sciences, Arlington Heights, Massachusetts, USA). The membranes were washed three time in TBS-T for ten minutes each and then processed for enhanced chemiluminescence detection according to the manufacturer's instructions (Amersham Life Science). Equal loading of samples was confirmed by probing for GAPDH.

mRNA analysis was performed as follows. RNA was extracted from cells using the RNA easy kit (Qiagen AG, Hombrechtikon, Switzerland). The concentration of total RNA was determined spectrophotometrically at 260 nm using a NanoDrop spectrophotometer (NanoDrop Technologies, Wilmington, Deleware, USA). One microgram of total RNA was reverse-transcribed using the Superscript First-Strand Synthesis System for real-time PCR kit (Invitrogen, Karlsruhe, Germany) according to the manufacturer's instructions. PCR amplification was performed using the Fast SYBR Green Master Mix on a StepOnePlus real-time PCR System (Applied Biosystems, Foster City, California, USA). Primer sets for BRD2, BRD3, and BRD4 (Table 2) were designed using the Primer3 software package (Rozen S, Skaletsky H: Primer3 on the WWW for general users and for biologist programmers. In: Misener S, Krawetz SA (Eds) Methods in Molecular Biology, Vol 132: Bioinformatics Methods and Protocols. Towota, New Jersey, USA; Humana Press, Inc., 2000, pp 365-386) and primer sets for c-MYC were from published studies. All samples were analyzed in triplicate. The relative quantity of the specific mRNA for each sample was calculated based on mean cycle threshold (Ct) values using the delta-delta Ct with a correction for experimental variations by normalization to the housekeeping gene GAPDH.

**Table 2. Sequences of Used Primers**

| | |
|---|---|
| BRD2-F | 5'-ACTTGGCCTGCATGACTACC-3' |
| BRD2-R | 5'-CTGTAGCTTTCGTGCCATTG-3' |
| BRD3-F | 5'-CAACCATCACTGCAAACGTC-3' |
| BRD3-R | 5'-GGGAGTGGTTGTGTCTGCTT-3' |
| BRD4-F | 5'-AGTCATCCAGCACCACCATT-3' |
| BRD4-R | 5'-TCTTAGGCTGGACGTTTTGC-3' |
| MYC-F | 5'-GGTGCTCCATGAGGAGACA-3' |
| MYC-R | 5'-CCTGCCTCTTTTCCACAGAA-3' |

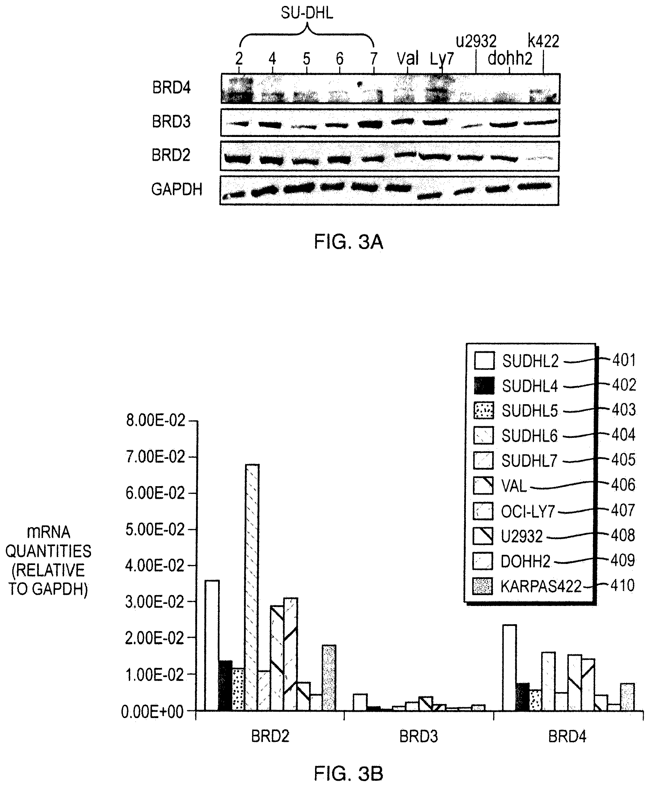

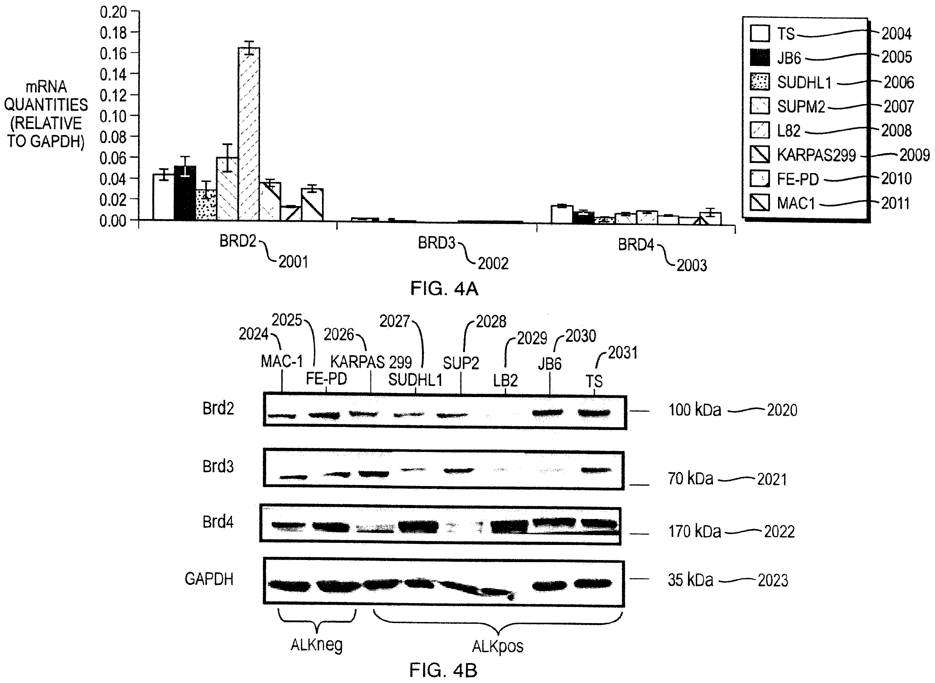

Results showed that basal levels of BRD2 mRNA and protein varied widely among DLBCL cell lines and ALCL cell lines, as illustrated in Figures 3 and 4, respectively, while all cell lines tested had low levels of BRD4 mRNA and protein and only trace levels of BRD3 mRNA and protein. Interestingly, basal levels of BRD2 mRNA and protein were not correlated with sensitivity to growth inhibition by Formula 2. Among the DLBCL lines tested, similar GI₅₀ values were obtained for the line with the highest BRD2 mRNA levels (SU-DHL-6 GI₅₀ = 110 nM) and lowest BRD2 mRNA levels (DoHH2 GI₅₀ = 90 nM). Similarly, among the ALCL lines tested similar GI₅₀ values were obtained for the line with the highest BRD2 mRNA levels (L82 GI₅₀ = 36 nM) and lowest BRD2 mRNA levels (FE-PD GI₅₀ = 158 nM).

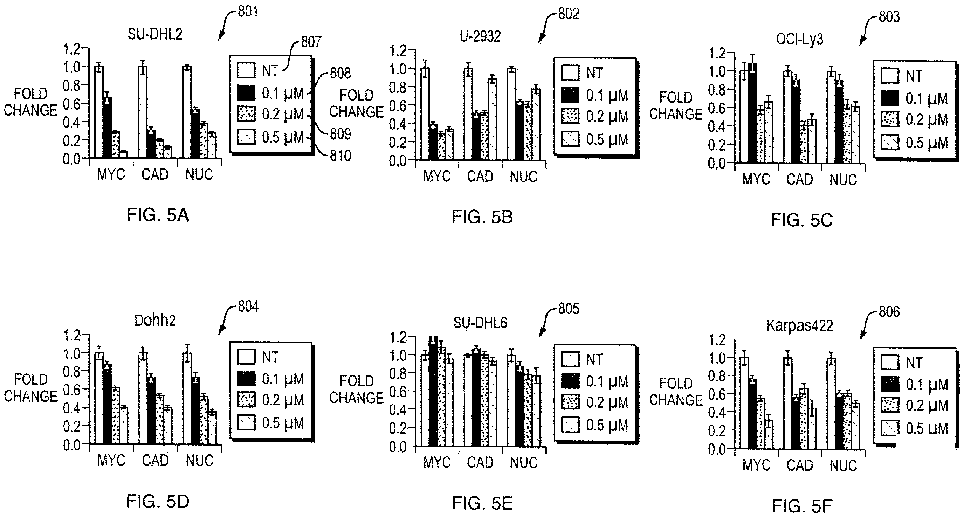

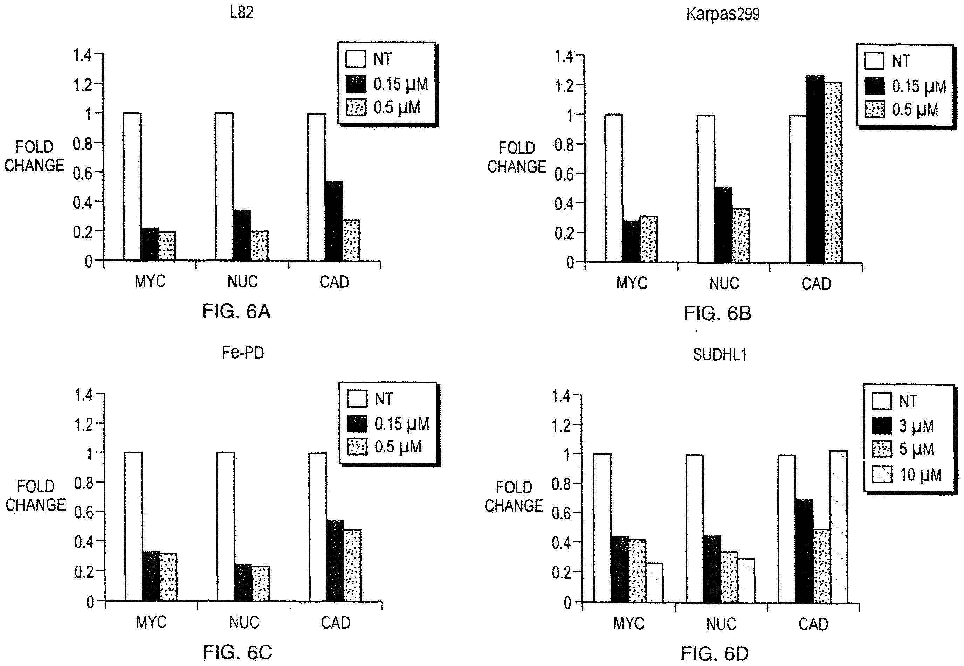

Results of mRNA analysis showed that 24 hour exposure to Formula 2 resulted in marked down-regulation of c-MYC mRNA in five of six (5 of 6) DLBCL cell lines tested and four of four (4 of 4) ALCL cell lines tested, as illustrated in Figures 5 and 6, respectively. Interestingly, the DLBCL line that showed minimal down-regulation of c-MYC was very sensitive growth inhibition by Formula 2 (SU-DHL-6 GI₅₀ = 110 nM).

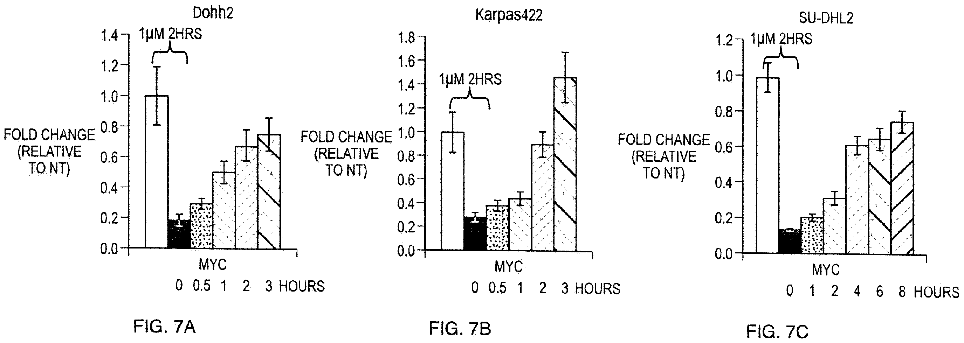

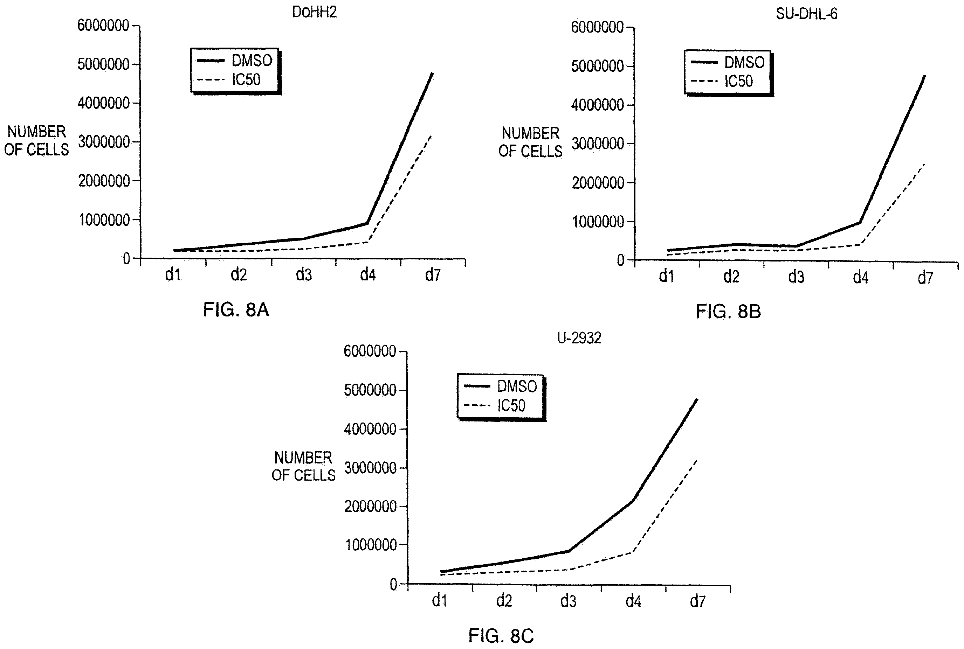

To evaluate whether the Formula 2-induced down-regulation of c-MYC was reversible, three DLBCL lines (DoHH2, Karpas 422, SU-DHL-2) were treated with Formula 2 for two hours and then the medium containing Formula 2 was changed to a medium not containing Formula 2 ("wash-out"). Following wash-out, a time-dependent restoration of c-MYC mRNA expression was observed in all three cell lines, with different kinetics, as illustrated in Figure 7. In a related experiment, DLBCL cells treated with Formula 2 at the GI₅₀ concentration for 24 hours started to regrow after "wash out" as illustrated in Figure 8.

### EXAMPLE 3: EFFECTS FOR FORMULA 2 ON DOWN-REGUATION OF NFκB

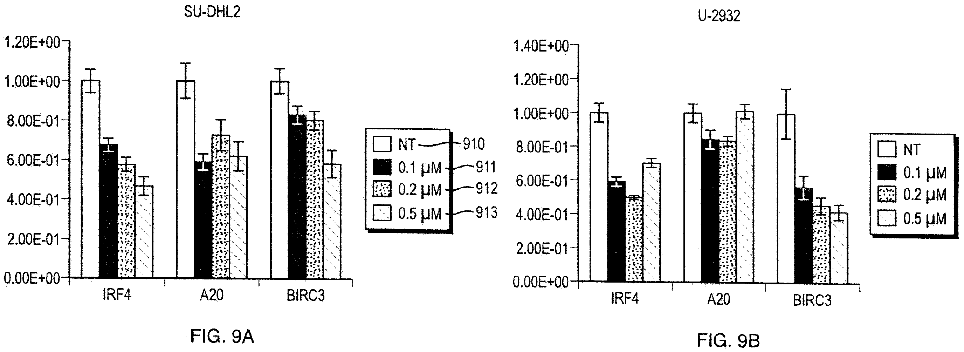

Formula 2 previously has been shown to be a competitive inhibitor of the BET bromodomain proteins 2, 3, and 4 (BRD2, BRD3, BRD4), as disclosed in U.S. Patent Appl. Publ. No. 20100286127, and BRD4 has been reported to be involved in the regulation of the transcription factor NFκB, which can act as a tumor suppressor in certain settings *[*Huang B, Yang XD, Zhou MM, Ozato K, Chen LF: Brd4 coactivates transcriptional activation of NF-κB via specific binding to acetylated RelA. Mol Cell Biol 2009; 29:1375-1387*].* Accordingly, effects of Formula 2 on mRNA expression of NFκB targets (IRF4, A20, BIRC3) were evaluated in five DLBCL (DoHH₂, Karpas 422, SU-DHL-2, SU-DHL-6, U2932) cell lines. Results showed that Formula 2 induced down-regulation of NFκB targets; representative results are shown in Figure 9.

## Claims

1. Composition for use in the treatment of B-cell malignant cancer or T-cell malignant cancer, wherein B-cell malignant cancer is diffuse large B-cell lymphoma or splenic marginal zone lymphoma and wherein T-cell malignant cancer is anaplastic large T-cell lymphoma, comprising a thienotriazolodiazepine compound, said thienotriazolodiazepine compound being represented by the following Formula (1): 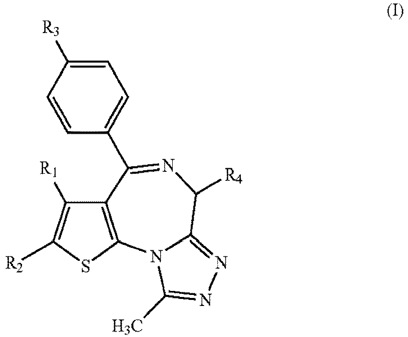 wherein R₁ is alkyl having a carbon number of 1-4, R₂ is a hydrogen atom; a halogen atom; or alkyl having a carbon number of 1-4 optionally substituted by a halogen atom or a hydroxyl group, R₃ is a halogen atom; phenyl optionally substituted by a halogen atom, alkyl having a carbon number of 1-4, alkoxy having a carbon number of 1-4 or cyano; --NR₅--(CH₂)ₘ--R₆ wherein R₅ is a hydrogen atom or alkyl having a carbon number of 1-4, m is an integer of 0-4, and R₆ is phenyl or pyridyl optionally substituted by a halogen atom; or --NR₇--CO--(CH₂)ₙ--R₈ wherein R₇ is a hydrogen atom or alkyl having a carbon number of 1-4, n is an integer of 0-2, and R₈ is phenyl or pyridyl optionally substituted by a halogen atom, and R₄ is --(CH₂)ₐ--CO--NH--R₉ wherein a is an integer of 1-4, and R₉ is alkyl having a carbon number of 1-4; hydroxyalkyl having a carbon number of 1-4; alkoxy having a carbon number of 1-4; or phenyl or pyridyl optionally substituted by alkyl having a carbon number of 1-4, alkoxy having a carbon number of 1-4, amino or a hydroxyl group or --(CH₂)_{b}-COOR₁₀ wherein b is an integer of 1-4, and R₁₀ is alkyl having a carbon number of 1-4, or a pharmaceutically acceptable salt thereof or a hydrate or solvate thereof.

2. The composition for use of claim 1, wherein the patient is a human.

3. The composition for use of claim 1, wherein the thienotriazolodiazepine compound represented by Formula 1 is independently selected from the group consisting of:
(i) (S)-2-[4-(4-chlorophenyl)-2,3,9-trimethyl-6H-thieno[3,2-f][1,2,4]triazolo-[4,3-a][1,4]diazepin-6-yl]-N-(4-hydroxyphenyl)acetamide or a dihydrate thereof, (ii) methyl (S)-{4-(3'-cyanobiphenyl-4-yl)-2,3,9-trimethyl-6H-thieno[3,2-f][1,2,4]tri-azolo[4,3-a][1,4]diazepin-6-yl}acetate, (iii) methyl (S)-{2,3,9-trimethyl-4-(4-phenylaminophenyl)-6H-thieno[3,2-f][1,2,4]triaz-olo[4,3-a][1,4]diazepin-6-yl}acetate; and (iv) methyl (S)-{2,3,9-trimethyl-4-[4-(3-phenylpropionylamino)phenyl]-6H-thieno[3,2-f-][1,2,4]triazolo[4,3-a][1,4]diazepin-6-yl} acetate.

4. The composition for use of claim 1, wherein the thienotriazolodiazepine compound is (S)-2-[4-(4-chlorophenyl)-2,3,9-trimethyl-6H-thieno[3,2-f][1,2,4]triazolo-[4,3-a][1,4]diazepin-6-yl]-N-(4-hydroxyphenyl)acetamide represented by the following Formula (2)

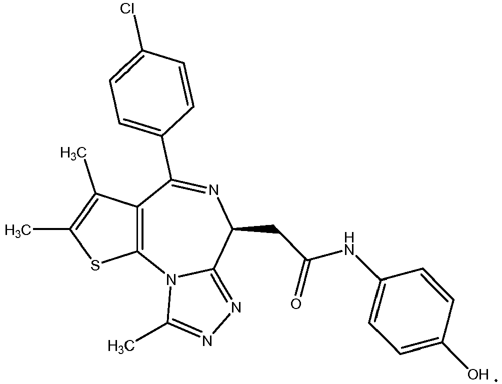

5. The composition for use of claim 4, wherein the patient is a human.

6. The composition for use of claim 4, wherein the B-cell malignancy is diffuse large B-cell lymphoma.

7. The composition for use of claim 4, wherein the B-cell malignant cancer is splenic marginal zone lymphoma.

8. The composition for use of claim 4, wherein the T-cell malignant cancer is anaplastic large T-cell lymphoma.

## Patentansprüche

1. Zusammensetzung zur Verwendung bei der Behandlung eines malignen B-Zell-Karzinoms oder malignen T-Zell-Karzinoms, wobei das maligne B-Zell-Karzinom diffuses großzelliges B-Zell-Lymphom oder splenisches Marginalzonenlymphom ist, und wobei das maligne T-Zell-Karzinom anaplastisches großzelliges T-Zell-Lymphom ist, umfassend eine Thienotriazolodiazepinverbindung, wobei die Thienotriazolodiazepinverbindung dargestellt wird durch die folgende Formel (1): 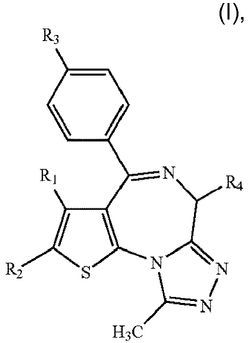 wobei R₁ ist: Alkyl mit einer Kohlenstoffzahl von 1-4, R₂ ist: ein Wasserstoffatom; ein Halogenatom; oder Alkyl mit einer Kohlenstoffzahl von 1-4, gegebenenfalls substituiert durch ein Halogenatom oder eine Hydroxylgruppe, R₃ ist: ein Halogenatom; Phenyl, das gegebenenfalls durch ein Halogenatom substituiert ist, Alkyl mit einer Kohlenstoffzahl von 1-4, Alkoxy mit einer Kohlenstoffzahl von 1-4 oder Cyano; --NR5--(CH₂)ₘ--R₆, wobei R₅ ein Wasserstoffatom oder Alkyl mit einer Kohlenstoffzahl von 1-4 ist, m eine ganze Zahl von 0-4 ist und R₆ Phenyl oder Pyridyl, gegebenenfalls substituiert durch ein Halogenatom, ist; oder --NR₇--CO-(CH₂)ₙ--R₈, wobei R₇ ein Wasserstoffatom oder Alkyl mit einer Kohlenstoffzahl von 1-4 ist, n eine ganze Zahl von 0-2 ist und R₈ Phenyl oder Pyridyl, gegebenenfalls substituiert durch ein Halogenatom, ist, und R₄ ist: --(CH₂)ₐ-CO-NH--R₉, wobei a eine ganze Zahl von 1-4 ist und Rg Alkyl mit einer Kohlenstoffzahl von 1-4 ist; Hydroxyalkyl mit einer Kohlenstoffzahl von 1-4; Alkoxy mit einer Kohlenstoffzahl von 1-4; oder Phenyl oder Pyridyl, gegebenenfalls substituiert durch Alkyl mit einer Kohlenstoffzahl von 1-4, Alkoxy mit einer Kohlenstoffzahl von 1-4, Amino oder eine Hydroxylgruppe oder --(CH₂)_{b}--COOR₁₀, wobei b eine ganze Zahl von 1-4 ist und R₁₀ Alkyl mit einer Kohlenstoffzahl von 1-4, ist, oder ein pharmazeutisch annehmbares Salz davon oder ein Hydrat oder Solvat davon.

2. Die Zusammensetzung zur Verwendung nach Anspruch 1, wobei der Patient ein Mensch ist.

3. Die Zusammensetzung zur Verwendung nach Anspruch 1, wobei die Thienotriazolodiazepinverbindung, die durch Formel 1 dargestellt wird, unabhängig ausgewählt ist aus der Gruppe bestehend aus:
(i) (S)-2-[4-(4-Chlorphenyl)-2,3,9-trimethyl-6H-thieno[3,2-f][1,2,4]triazolo-[4,3-a]-[1,4]diazepin-6-yl]-N-(4-hydroxyphenyl)acetamid oder einem Dihydrat davon, (ii) Methyl-(S)-{4-(3'-cyanobiphenyl-4-yl)-2,3,9-trimethyl-6H-thieno[3,2-f][1,2,4]-triazolo[4,3-a][1,4]diazepin-6-yl}acetat, (iii) Methyl-(S)-{2,3,9-trimethyl-4-(4-phenylaminophenyl)-6H-thieno[3,2-f][1,2,4]triazolo[4,3-a][1,4]diazepin-6-yl}acetat und (iv) Methyl-(S)-{2,3,9-trimethyl-4-[4-(3-phenylpropionylamino)phenyl]-6H-thieno[3,2-f][1,2,4]triazolo[4,3-a][1,4]diazepin-6-yl}acetat.

4. Die Zusammensetzung zur Verwendung nach Anspruch 1, wobei die Thienotriazolodiazepinverbindung (S)-2-[4-(4-Chlorphenyl)-2,3,9-trimethyl-6H-thieno[3,2-f][1,2,4]triazolo-[4,3-a][1,4]diazepin-6-yl]-N-(4-hydroxyphenyl)acetamid ist, dargestellt durch die folgende Formel (2)

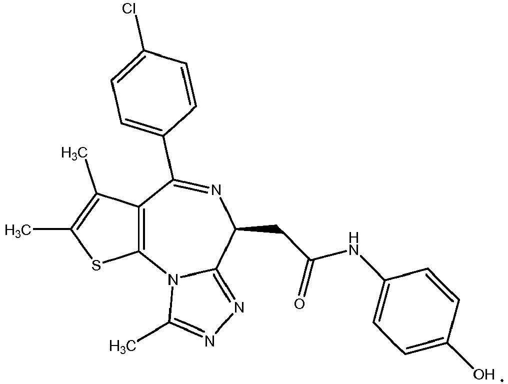

5. Die Zusammensetzung zur Verwendung nach Anspruch 4, wobei der Patient ein Mensch ist.

6. Die Zusammensetzung zur Verwendung nach Anspruch 4, wobei die B-Zell-Malignität diffuses großzelliges B-Zell-Lymphom ist.

7. Die Zusammensetzung zur Verwendung nach Anspruch 4, wobei das maligne B-Zell-Karzinom splenisches Marginalzonenlymphom ist.

8. Die Zusammensetzung zur Verwendung nach Anspruch 4, wobei das maligne T-Zell-Karzinom anaplastisches großzelliges T-Zell-Lymphom ist.

## Revendications

1. Composition pour une utilisation dans le traitement d'un cancer malin des cellules B ou d'un cancer malin des cellules T, où le cancer malin des cellules B est un lymphome des cellules B large diffus ou un lymphome de zone marginale splénique et où le cancer malin des cellules T est un lymphome des cellules T large anaplasique, comprenant un composé de thiénotriazolodiazépine, ledit composé de thiénotriazolodiazépine étant représenté par la formule (1) suivante: 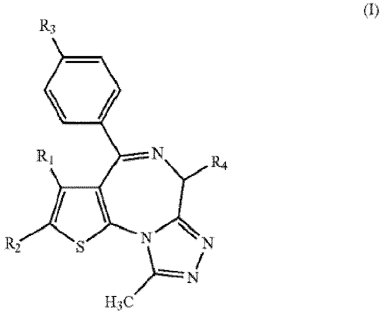 dans laquelle R₁ est un alkyle possédant un nombre de carbones de 1-4, R₂ est un atome d'hydrogène; un atome d'halogène; ou un alkyle possédant un nombre de carbones de 1-4 optionnellement substitué par un atome d'halogène ou un groupe hydroxyle, R₃ est un atome d'halogène; un phényle optionnellement substitué par un atome d'halogène, un alkyle possédant un nombre de carbones de 1-4, un alcoxy possédant un nombre de carbones de 1-4 ou un cyano; -NR₅-(CH₂)ₘ-R₆ où R₅ est un atome d'hydrogène ou un alkyle possédant un nombre de carbones de 1-4, m est un nombre entier de 0-4 et R₆ est un phényle ou un pyridyle optionnellement substitué par un atome d'halogène; ou -NR₇-CO-(CH₂)ₙ-R₈ où R₇ est un atome d'hydrogène ou un alkyle possédant un nombre de carbones de 1-4, n est un nombre entier de 0-2 et R₈ est un phényle ou un pyridyle optionnellement substitué par un atome d'halogène, et R₄ est -(CH₂)ₐ-CO-NH-R₉ où a est un nombre entier de 1-4 et R₉ est un alkyle possédant un nombre de carbones de 1-4; un hydroxyalkyle possédant un nombre de carbones de 1-4; un alcoxy possédant un nombre de carbones de 1-4; ou un phényle ou un pyridyle optionnellement substitué par un alkyle possédant un nombre de carbones de 1-4, un alcoxy possédant un nombre de carbones de 1-4, un amino ou un groupe hydroxyle ou -(CH₂)_{b}-COOR₁₀ où b est un nombre entier de 1-4 et R₁₀ est un alkyle possédant un nombre de carbones de 1-4, ou un sel pharmaceutiquement acceptable de celui-ci ou un hydrate ou un solvate de celui-ci.

2. Composition pour une utilisation selon la revendication 1, où le patient est un humain.

3. Composition pour une utilisation selon la revendication 1, dans laquelle le composé de thiénotriazolodiazépine représenté par la formule 1 est indépendamment choisi dans le groupe constitué de:
(i) (S)-2-[4-(4-chlorophényl)-2,3,9-triméthyl-6H-thiéno[3,2-f][1,2,4]triazolo[4,3-a][1,4]diazépin-6-yl]-N-(4-hydroxyphényl)acétamide ou un dihydrate de celui-ci, (ii) (S)-{4-(3'-cyanobiphényl-4-yl)-2,3,9-triméthyl-6H-thiéno[3,2-f][1,2,4]triazolo[4,3-a][1,4]diazépin-6-yl}acétate de méthyle, (iii) (S)-{2,3,9-triméthyl-4-(4-phénylaminophényl)-6H-thiéno[3,2-f][1,2,4]triazolo[4,3-a][1,4]diazépin-6-yl} acétate de méthyle, et (iv) (S)-{2,3,9-triméthyl-4-[4-(3-phénylpropionylammo)phényl]-6H-thiéno[3,2-f][1,2,4]triazolo[4,3-a][1,4]diazépin-6-yl} acétate de méthyle.

4. Composition pour une utilisation selon la revendication 1, dans laquelle le composé de thiénotriazolodiazépine est le (S)-2-[4-(4-chlorophényl)-2,3,9-triméthyl-6H-thiéno[3,2-f][1,2,4]triazolo[4,3-a][1,4]diazépin-6-yl]-N-(4-hydroxyphényl)acétamide représenté par la formule (2) suivante:

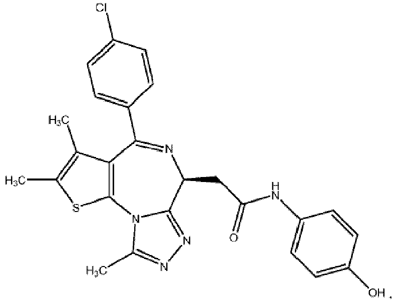

5. Composition pour une utilisation selon la revendication 4, où le patient est un humain.

6. Composition pour une utilisation selon la revendication 4, où la malignité des cellules B est un lymphome des cellules B large diffus.

7. Composition pour une utilisation selon la revendication 4, où le cancer malin des cellules B est un lymphome de zone marginale splénique.

8. Composition pour une utilisation selon la revendication 4, où le cancer malin des cellules T est un lymphome des cellules T large anaplasique.


### SEQUENCE LISTING

<110> Oncoethix
<120> Method of treating lymphoma using thienotriazolodiazepine compounds
<130> M/MGN-031-PC
<160> 8
<170> BiSSAP 1.2
<210> 1
   <211> 20
   <212> DNA
   <213> Artificial Sequence
<220>
   <221> source
   <222> 1..20
   <223> /organism="Artificial Sequence" /note="Forward primer for BET bromodomain protein 2" /mol_type="unassigned DNA"
<400> 1
   acttggcctg catgactacc 20
<210> 2
   <211> 20
   <212> DNA
   <213> Artificial Sequence
<220>
   <221> source
   <222> 1..20
   <223> /organism="Artificial Sequence" /note="Reverse primer for BET bromodomain protein 2" /mol_type="unassigned DNA"
<400> 2
   ctgtagcttt cgtgccattg 20
<210> 3
   <211> 20
   <212> DNA
   <213> Artificial Sequence
<220>
   <221> source
   <222> 1..20
   <223> /organism="Artificial Sequence" /note="Forward primer for BET bromodomain protein 3" /mol_type="unassigned DNA"
<400> 3
   caaccatcac tgcaaacgtc 20
<210> 4
   <211> 20
   <212> DNA
   <213> Artificial Sequence
<220>
   <221> source
   <222> 1..20
   <223> /organism="Artificial Sequence" /note="Reverse primer for BET bromodomain protein 3" /mol_type="unassigned DNA"
<400> 4
   gggagtggtt gtgtctgctt 20
<210> 5
   <211> 20
   <212> DNA
   <213> Artificial Sequence
<220>
   <221> source
   <222> 1..20
   <223> /organism="Artificial Sequence" /note="Forward primer for BET bromodomain protein 4" /mol_type="unassigned DNA"
<400> 5
   agtcatccag caccaccatt 20
<210> 6
   <211> 20
   <212> DNA
   <213> Artificial Sequence
<220>
   <221> source
   <222> 1..20
   <223> /organism="Artificial Sequence" /note="Reverse primer for BET bromodomain protein 4" /mol_type="unassigned DNA"
<400> 6
   tcttaggctg gacgttttgc 20
<210> 7
   <211> 19
   <212> DNA
   <213> Artificial Sequence
<220>
   <221> source
   <222> 1..19
   <223> /organism="Artificial Sequence" /note="Forward primer for c-MYC oncogene" /mol_type="unassigned DNA"
<400> 7
   ggtgctccat gaggagaca 19
<210> 8
   <211> 20
   <212> DNA
   <213> Artificial Sequence
<220>
   <221> source
   <222> 1..20
   <223> /organism="Artificial Sequence" /note="Reverse primer for c-MYC oncogene" /mol_type="unassigned DNA"
<400> 8
   cctgcctctt ttccacagaa 20